# FASCICULE DE BREVET EUROPEEN

(11) **EP 0 666 113 B1**
(45) Date de publication et mention de la délivrance du brevet: **19.03.1997**
(21) Numéro de dépôt: 95400119.4
(22) Date de dépôt: 20.01.1995
(51) Int. Cl.: B06B 3/00, G10K 11/02

(54) **Dispositif de percussion à ultrasons**
Ultraschallperkussionsgerät
Ultrasonic percussion device

(30) Priorité: 03.02.1994 FR 9401201
(43) Date de publication de la demande: 09.08.1995
(73) Titulaire: AEROSPATIALE Société Nationale Industrielle, 75781 Paris Cédex 16 (FR)
(72) Inventeur: Kremer, Daniel, F-77380 Combes La Ville (FR); Drobinski, Gérard, 92260 Fontenay aux Roses (FR)
(74) Mandataire: Bonnetat, Christian

(56) Documents cités:
- EP-A- 0 424 231
- US-A- 5 243 997

## Description

La présente invention concerne un dispositif de percussion à ultrasons plus particulièrement destiné, quoique non exclusivement, à des applications médicales relatives aux traitements de canaux corporels ou conduits morphologiques, tels que par exemple la destruction d'athéromes à l'intérieur des artères. Bien évidemment, il va de soi que le dispositif de percussion pourrait être utilisé pour d'autres applications notamment industrielles, telles que l'usinage, le polissage, le débouchage de conduits, etc..., sans sortir du cadre de l'invention.

On sait que, pour détruire des athéromes, l'une des techniques disponibles aux praticiens consiste à utiliser des dispositifs de percussion à ultrasons qui comprennent généralement :
- un générateur d'ondes ultrasonores, terminé par un organe transmettant les vibrations engendrées ;
- un fil, dont l'extrémité proximale est portée par ledit organe et dont l'extrémité distale est animée d'un mouvement de percussion ; et
- des moyens de liaison pour immobiliser l'extrémité proximale dudit fil dans ledit organe du générateur.

Ainsi, par l'intermédiaire du générateur, les vibrations engendrées sont transmises au fil qui est introduit par le praticien dans le canal corporel à traiter et dont l'extrémité distale martèle cycliquement l'athérome à détruire.

Comme le souligne le préambule du brevet français 2 653 040, l'inconvénient principal de ces dispositifs concerne leurs moyens de liaison qui n'assurent pas une transmission maximale des vibrations au fil, en raison de son diamètre millimétrique et des jeux fonctionnels qui en résultent. Aussi, l'invention décrite dans le brevet consiste à remédier à cela en proposant des moyens de liaison qui comprennent :
- un manchon radialement déformable de façon élastique, recevant l'extrémité proximale dudit fil et pourvu d'une partie évasée conique ;
- un perçage prévu dans ledit organe de transmission et dans lequel est introduit ledit manchon, la partie conique dudit manchon venant au contact d'une paroi conique complémentaire ménagée dans ledit perçage ; et
- un élément de serrage coopérant par vissage avec ledit organe et agissant contre ledit manchon, pour que, lors du vissage, la partie conique dudit manchon soit pressée contre la paroi conique dudit perçage en entraînant, par la déformation radiale dudit manchon, le serrage de l'extrémité proximale dudit fil, jusqu'à ce que ledit élément de serrage vienne au contact d'une butée prévue sur ledit organe.

Bien que les dispositifs de percussion équipés de ces moyens de liaison donnent des résultats efficaces en ce qui concerne le traitement proprement dit, c'est-à-dire la destruction des athéromes, il s'avère néanmoins peu commode à l'usage en ce qui concerne leur manipulation. En effet, d'une part, il est nécessaire de visser fortement l'élément de serrage (l'écrou) sur ledit organe par l'intermédiaire de clés annexes, ce qui n'est pas apprécié des praticiens. D'autre part, il est apparu des coincements entre le manchon et l'extrémité proximale du fil, qui empêchent parfois leur démontage manuellement, à cause principalement des défauts des surfaces et au matage inévitable de celles-ci.

La présente invention a pour but de remédier à ces inconvénients et concerne un dispositif de percussion à ultrasons, dont les moyens de liaison permettent de maintenir le fil sur ledit organe sans avoir recours à un serrage important, ni à des outils annexes, et tout en assurant une transmission maximale desdites vibrations.

A cet effet, le dispositif de percussion à ultrasons, du type décrit préalablement est remarquable, selon l'invention, en ce que ledit organe est réalisé en une matière élastiquement déformable et en ce que lesdits moyens de liaison sont ménagés sur ledit organe de façon à faire partie intégrante de celui-ci, lesdits moyens de liaison étant susceptibles d'occuper une position spontanément rapprochée, pour laquelle l'extrémité proximale dudit fil est serrée par ledit organe, et une position volontairement écartée par des moyens d'actionnement, pour laquelle l'extrémité proximale dudit fil peut être libérée dudit organe ou engagée dans ledit organe.

Ainsi, grâce à sa conception en une matière élastiquement déformable, ledit organe terminant le générateur d'ondes ultrasonores, peut être directement utilisé comme moyens de liaison, ce qui permet de s'affranchir notamment des pièces usuelles qui les constituent (élément de serrage et manchon précédemment employés), pour fixer le fil au dispositif, et des inconvénients qu'elles engendrent comme il est rappelé ci-dessus.

De préférence, lesdits moyens de liaison font partie de l'extrémité libre dudit organe, opposée à son autre extrémité reliée audit générateur, et ils sont par exemple définis par un passage, ménagé dans ladite extrémité libre dudit organe depuis sa face transversale, et apte à recevoir l'extrémité proximale dudit fil, et par au moins une fente longitudinale pratiquée en bout de ladite extrémité libre, depuis sa surface latérale externe jusqu'audit passage, pour la conformer en extrémité fendue élastiquement déformable. Ainsi, la fente contribue à rendre élastiquement déformable l'extrémité libre dudit organe, laquelle constitue des mors de préhension pour serrer, avec une force constante, l'extrémité proximale du fil, par la surface latérale interne délimitant le passage.

Pour cela, ledit passage présente initialement un diamètre inférieur au diamètre dudit fil, de façon que, lorsque lesdits moyens d'actionnement sont commandés, lesdits moyens de liaison à extrémité fendue occupent la position volontairement écartée pour permettre l'introduction dudit fil dans ledit passage présentant alors un diamètre supérieur à celui dudit fil, et lorsque lesdits moyens d'actionnement sont relâchés, lesdits moyens de liaison occupent la position spontanément rapprochée, pour permettre le serrage par pincement de l'extrémité proximale du fil.

Plus particulièrement, ledit organe présente une forme cylindrique de révolution, et ledit passage est situé axialement dans ledit organe, depuis la face transversale de son extrémité libre, tandis que ladite fente longitudinale débouche au droit dudit passage. De préférence, ladite fente longitudinale traverse de part en part l'extrémité libre dudit organe en passant par ledit passage, pour la séparer en deux parties alors identiques. Bien entendu, on pourrait également ménager deux fentes longitudinales diamétrales, perpendiculaires entre elles, ou plus, pour séparer l'extrémité libre en quatre parties identiques, ou alors trois fentes longitudinales radiales, régulièrement espacées de 120° les unes par rapport aux autres, pour séparer l'extrémité libre en trois parties identiques.

Selon une autre caractéristique de l'invention, le dispositif comprend, de plus, un corps creux entourant ledit organe et portant, avantageusement, à l'une de ses extrémités à l'aplomb desdits moyens de liaison, lesdits moyens d'actionnement. Ce corps permet en outre au praticien de manipuler le dispositif.

Par exemple, lesdits moyens d'actionnement peuvent comprendre au moins une pièce d'écartement déplaçable qui est logée dans un trou prévu radialement dans l'extrémité correspondante dudit corps creux, et qui fait face à ladite fente, ladite pièce étant susceptible de pénétrer dans ladite fente pour écarter les faces latérales qui la délimitent et agrandir ledit passage, lorsqu'elle est actionnée.

De préférence, lorsque ladite fente longitudinale traverse diamétralement de part en part l'extrémité libre dudit organe, lesdits moyens d'actionnement comprennent, en regard des bords latéraux respectivement opposés délimitant ladite fente, une pièce d'écartement déplaçable et une pièce d'écartement fixe, lesdites pièces étant logées respectivement dans des trous radiaux opposés, prévus dans l'extrémité correspondante dudit corps creux. Les deux pièces pourraient être également déplaçables.

Avantageusement, entre l'extrémité libre dudit organe et l'extrémité correspondante dudit corps, est prévu un manchon d'amortissement, en élastomère ou analogue, traversé par la ou lesdites pièces d'écartement. De la sorte, on évite les vibrations de l'ensemble, le corps étant isolé de l'organe. En particulier, ladite pièce d'écartement est radialement déplaçable en direction de la fente par une vis de pression qui est logée dans ledit corps pour agir sur ladite pièce, et qui est commandable par une poignée ou analogue extérieure audit corps.

Ainsi, l'actionnement des moyens de liaison s'effectue aisément par le praticien qui peut agir sur la poignée des moyens d'écartement tout en maintenant le corps du dispositif de percussion, sans avoir à recourir à des outils annexes.

Par ailleurs, la ou lesdites pièces d'écartement sont terminées par des pointes coniques susceptibles de s'engager dans des empreintes sensiblement complémentaires ménagées dans les bords latéraux délimitant ladite fente. L'écartement desdites parties de l'extrémité libre fendue est ainsi facilité. Aussi, autour de la ou desdites pièces d'écartement, sont disposés des joints toriques ou analogues entre celles-ci et la surface latérale externe de ladite extrémité libre dudit organe. Ces joints toriques jouent le rôle de ressort de rappel pour lesdites pièces après leur actionnement, évitant le contact des pointes coniques avec l'organe au cours du fonctionnement de celui-ci.

Les figures du dessin annexé feront bien comprendre comment l'invention peut être réalisée. Sur ces figures, des références identiques désignent des éléments semblables.

La figure 1 représente schématiquement un dispositif de percussion à ultrasons conforme à l'invention.

La figure 2 montre, en coupe axiale, un exemple de réalisation de l'organe dudit générateur avant le montage du fil.

La figure 3 est une vue en coupe selon la ligne III-III de la figure 2.

La figure 4 montre, en coupe axiale partielle agrandie, les moyens de liaison dudit organe, en position écartée, pour le montage dudit fil.

La figure 5 est une vue en coupe selon la ligne V-V de la figure 4.

La figure 6 montre, en coupe axiale partielle agrandie, les moyens de liaison dudit organe, en position spontanément rapprochée, pour le serrage dudit fil.

La figure 7 est une vue en coupe selon la ligne VII-VII de la figure 6.

La figure 8 illustre schématiquement l'action de l'extrémité distale dudit fil sur l'athérome à détruire.

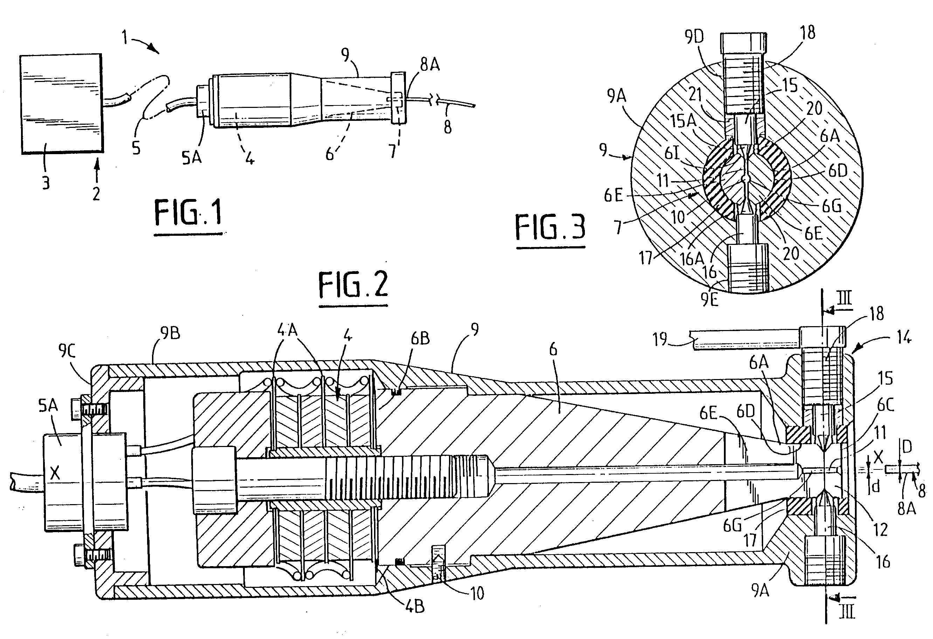

Le dispositif de percussion à ultrasons 1, représenté sur la figure 1, comprend un générateur d'ondes ultrasonores 2 d'un type connu en soi et comportant un générateur électrique à haute fréquence 3 et un convertisseur piézo-électrique 4 reliés entre eux par une liaison électrique 5. Un organe 6 de transmission des vibrations produites est associé au convertisseur 4 et il porte, par l'intermédiaire de moyens de liaison 7, l'extrémité proximale 8A d'un fil 8, dont l'extrémité distale 8B est susceptible d'être introduite, dans cette application préférentielle, dans un canal corporel à traiter, pour venir au contact d'un dépôt à détruire, tel qu'une formation athéromateuse par exemple. La destruction de celle-ci est obtenue à partir du générateur 2 qui convertit l'énergie électrique produite en énergie mécanique, par ledit convertisseur, qui est transmise par ledit organe 6 le long dudit fil 8 sous forme de vibrations mécaniques, dont l'extrémité distale 8B martèle alors cycliquement la formation athéromateuse.

Pour transmettre intégralement cette énergie mécanique vibratoire au fil, l'organe de transmission 6 est réalisé en une matière élastiquement déformable et les moyens de liaison 7 sont avantageusement intégrés à l'organe 6. Comme le montre la figure 2, l'organe 6 a une forme cylindrique de révolution, d'axe longitudinal X-X et son extrémité libre ou aval 6A définit alors les moyens de liaison de l'extrémité proximale 8A du fil. A l'extrémité opposée amont 6B de l'organe est associé le convertisseur piézo-électrique 4 qui comporte notamment des rondelles d'alimentation 4A et au moins un isolant 4B. L'ensemble constitué de l'organe 6 et du convertisseur 4 est logé dans un corps cylindrique creux 9, rendu solidaire de l'organe par des vis 10 et permettant au praticien de manipuler le dispositif 1.

Plus particulièrement, l'organe 6 présente une certaine conicité en direction de son extrémité libre 6A dans laquelle sont ménagés un passage 11, depuis sa face transversale 6C, et une fente longitudinale 12 qui, dans cet exemple de réalisation, traverse de part en part l'extrémité libre 6A en passant au milieu par le passage 11. Comme on peut le voir sur les figures 2 et 3, cette fente longitudinale 12 débouche diamétralement de la surface latérale externe 6D de l'extrémité libre 6A, et sépare celle-ci en deux parties identiques 6E, dont la section est demi-circulaire. La fente longitudinale 12 présente un écartement, compris entre les faces parallèles en regard 6F des parties identiques 6E, inférieur au diamètre d du passage 11 dont la surface latérale interne 6G délimitée par les deux parties 6E est destinée à maintenir par pincement l'extrémité proximale 8A du fil, comme on le verra ultérieurement. Ce diamètre d est initialement inférieur au diamètre D du fil qui peut avoir, selon les types de canaux et de conduits à traiter, une valeur comprise entre 0,2 mm et 0,5 mm. Sur les figures, les dimensions du passage et du fil ont été exagérées pour des raisons de clarté des figures.

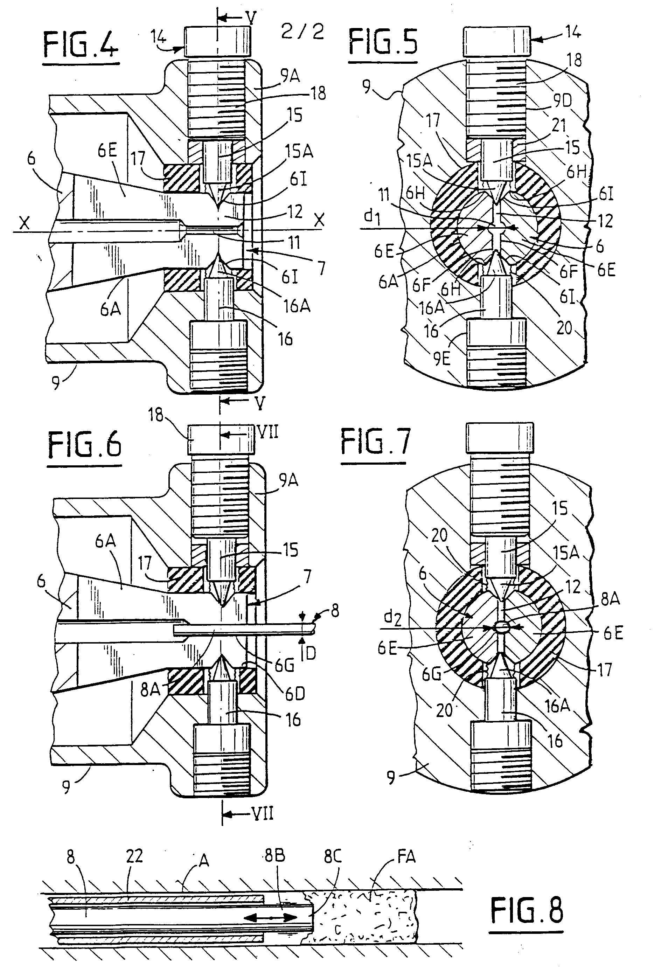

On comprend donc, que le passage axial 11, la fente longitudinale 12 et les parties 6E de l'extrémité libre 6A dudit organe, qui en résultent, forment les moyens de liaison intégrés 7 pour permettre le serrage par pincement, selon un effort constant, de l'extrémité proximale 8A du fil, par déformation élastique de ladite extrémité libre fendue dudit organe. En particulier, les parties 6E des moyens de liaison 7 peuvent occuper une position spontanément rapprochée, pour laquelle l'extrémité proximale du fil est serrée par l'organe 6 (figures 6 et 7), et une position volontairement écartée, par l'intermédiaire de moyens d'actionnement 14, pour laquelle l'extrémité proximale du fil peut être libérée de l'organe ou engagée dans celui-ci (figures 4 et 5).

Par ailleurs, le corps creux cylindrique 9, entourant l'organe de transmission 6 et le convertisseur piézo-électrique 4, porte, à son extrémité 9A située autour de l'extrémité libre 6A dudit organe, les moyens d'actionnement 14, et à son autre extrémité 9B, un couvercle 9C dans lequel est connectée la prise 5A de la liaison électrique 5 reliant le générateur 3 au convertisseur 4.

Structurellement, les moyens d'actionnement 14 sont définis, dans cet exemple de réalisation, par deux pièces d'écartement cylindriques 15 et 16 qui sont logées respectivement dans des trous radiaux et diamétralement opposés 9D et 9E, prévus dans l'extrémité 9A du corps 9, et qui sont situés en regard de la fente longitudinale 12, respectivement de part et d'autre de celle-ci. Plus particulièrement, entre l'extrémité 9A du corps et l'extrémité libre 6A de l'organe, est disposé un manchon d'amortissement 17 réalisé en élastomère et de section alors annulaire. Ce manchon 17 est traversé radialement par les pièces d'écartement, la liaison souple ainsi créée entre les deux extrémités de l'organe et du corps évite la vibration de l'ensemble. La pièce 15 est par exemple radialement déplaçable en direction de la fente 12, tandis que la pièce 16 peut être montée fixe dans le trou 9E. Pour faciliter l'engagement des pièces d'écartement dans la fente, entre les bords latéraux 6H qui la délimitent latéralement, des pointes coniques 15A et 16A terminent respectivement les pièces 15 et 16 pour se trouver à proximité d'empreintes coniques 6I, qui sont ménagées dans les bords latéraux 6H de l'extrémité 6A et dans lesquelles sont agencées lesdites pointes coniques. La pièce d'écartement 15 est rendue mobile par l'intermédiaire d'une vis de pression 18 qui est logée dans le trou 9D alors taraudé à ce niveau pour agir sur la pièce 15. Cette vis 18 est commandable par une poignée 19, à laquelle elle est liée et qui est avantageusement accessible extérieurement audit corps 9. Cette réalisation des moyens d'actionnement 14 est particulièrement avantageuse puisque le praticien, portant le corps 9, peut aisément procéder au montage et/ou au démontage de l'extrémité proximale d'un fil en manoeuvrant directement la poignée 19. Quant à l'autre pièce d'écartement 16, elle est vissée directement dans le trou taraudé 9E pour y être immobilisée, de façon que sa pointe conique 16A soit réglée à proximité de l'empreinte conique 6I.

En outre, des joints toriques 20 ou analogues sont prévus autour des pointes coniques 15A et 16A des pièces d'écartement, entre la surface latérale externe 6D de l'extrémité libre 6A dudit organe et les pièces d'écartement 15 et 16. La pièce mobile d'écartement 15 est, par ailleurs, logée dans le trou 9D du corps par l'intermédiaire d'une bague de guidage 21.

La mise en place de l'extrémité proximale 8A d'un fil 8 à l'intérieur de l'organe de transmission 6 du dispositif 1 se déroule de la façon suivante. Tout d'abord, les moyens de liaison 7, situés à l'extrémité libre 6A de l'organe de transmission 6, occupent la position initiale illustrée sur les figures 2 et 3, pour laquelle le diamètre d du passage est minimal, inférieur au diamètre D du fil.

Pour procéder à l'introduction de l'extrémité proximale du fil, le praticien agit sur la poignée 19 des moyens d'actionnement 14, laquelle, par l'intermédiaire de la vis 18, déplace radialement par rapport à l'axe X-X la pièce d'écartement 15, dont la pointe conique 15A s'applique sur l'empreinte conique correspondante 6I pour ouvrir la fente 12, par écartement des parties identiques 6E de l'extrémité fendue 6A. L'action radiale de la pièce d'écartement 15 produit, par l'intermédiaire du manchon élastique 17, un léger déplacement de l'extrémité 6A dans le même sens, ce qui a pour effet d'engager l'empreinte conique opposée 6I dans la pointe conique 16A de la pièce fixe 16 et, donc, de participer à l'écartement des parties 6E de l'organe. Comme le montrent les figures 4 et 5, les moyens de liaison 7 occupent alors une position volontairement écartée sous l'action des moyens 14, pour laquelle le passage a un diamètre d1 supérieur au diamètre D du fil par la déformation élastique des parties 6E de l'extrémité libre dudit organe, les pointes coniques écartant les faces en regard 6F de ladite fente.

A ce moment, l'extrémité proximale 8A du fil est introduite dans le passage 11 alors écarté, jusqu'à l'endroit souhaité, puis les moyens d'actionnement 14 sont relâchés, ce qui a pour effet d'éloigner radialement les pièces d'écartement 15 et 16 des bords latéraux 6H de la fente 12. Simultanément, les parties 6E de l'organe de transmission, c'est-à-dire les moyens de liaison 7, se rapprochent l'une de l'autre pour occuper la position spontanément rapprochée, de sorte que la surface interne 6G du passage 11 joue le rôle de mors de préhension en serrant par pincement l'extrémité proximale 8A du fil avec une force constante, comme le montrent les figures 6 et 7. Dans cette position, le diamètre d2 du passage est égal au diamètre D du fil et il est proche du diamètre initial d.

Les avantages procurés par la réalisation des moyens de liaison intégrés à l'extrémité libre élastiquement déformable dudit organe, concernent la constance de l'effort de serrage puisqu'il est indépendant du praticien, la manipulation plus aisée par les moyens d'actionnement, l'absence de blocage du fil dans l'organe et la conception plus simple desdits moyens de liaison.

On a représenté schématiquement sur la figure 8 une partie d'une artère A obstruée, par exemple, par une formation athéromateuse FA.

Pour procéder à l'élimination de cette formation, le praticien introduit préalablement une gaine 22 dans l'artère A jusqu'au voisinage de la formation athéromateuse FA. Cette gaine 22 assure le guidage du fil 8 dans l'artère en protégeant sa paroi interne des frottements du fil, qui pourraient occasionner des échauffements et des brûlures néfastes de l'artère.

Lorsque la face transversale 8C de l'extrémité distale 8B du fil est sensiblement au contact de la formation athéromateuse FA, le dispositif à ultrasons 1 selon l'invention est mis en action, les vibrations engendrées étant transmises de façon optimale au fil 8 grâce aux moyens de liaison 7 préalablement décrits et intégrés à l'organe de transmission 6.

Les vibrations du fil transmises longitudinalement, agissent contre la formation athéromateuse selon un mouvement alternatif (double flèche). Lors du mouvement d'avance du fil, la face 8C de l'extrémité distale 8B heurte la formation FA pour provoquer progressivement l'effritement et la fragmentation de celle-ci. En revanche, lors du mouvement de recul du fil, les particules de la formation athéromateuse sont enlevées progressivement de l'athérome par des effets de dépression ou de cavitation.

A titre d'exemple, l'organe 6 peut être réalisé en un acier approprié élastiquement déformable, tandis que le corps 9 est réalisé en une matière de préférence électriquement isolante, telle qu'un chlorure de polyvinyle.

## Revendications

1. Dispositif de percussion à ultrasons, du type comportant :
- un générateur d'ondes ultrasonores (2), terminé par un organe (6) transmettant les vibrations engendrées ;
- un fil (8), dont l'extrémité proximale est portée par ledit organe et dont l'extrémité distale est animée d'un mouvement de percussion ; et
- des moyens de liaison (7) pour immobiliser l'extrémité proximale dudit fil dans ledit organe du générateur,
caractérisé en ce que lesdits moyens de liaison (7) font partie intégrante dudit organe (6), en ce que ledit organe (6) et lesdits moyens de liaison (7) sont réalisés en une matière élastiquement déformable et en ce que lesdits moyens de liaison (7) sont susceptibles d'occuper une position spontanément rapprochée sous l'action de l'élasticité propre de ladite matière élastiquement déformable, position rapprochée pour laquelle l'extrémité proximale (8A) dudit fil (8) est serrée par ledit organe, et une position volontairement écartée par des moyens d'actionnement (14), à l'encontre de l'action de l'élasticité propre de ladite matière élastiquement déformable, position écartée pour laquelle l'extrémité proximale (8A) dudit fil peut être libérée dudit organe ou engagée dans ledit organe.

2. Dispositif selon la revendication 1,
caractérisé en ce que lesdits moyens de liaison (7) font partie de l'extrémité libre (6A) dudit organe, opposée à son autre extrémité reliée audit générateur, et ils sont définis par un passage (11), ménagé dans ladite extrémité libre dudit organe depuis sa face transversale, et apte à recevoir l'extrémité proximale dudit fil, et par au moins une fente longitudinale (12) pratiquée en bout de ladite extrémité libre, depuis sa surface latérale externe (6D) jusqu'audit passage (11), pour la conformer en extrémité fendue élastiquement déformable.

3. Dispositif selon la revendication 2,
caractérisé en ce que ledit passage (11) présente initialement un diamètre inférieur au diamètre dudit fil, de façon que, lorsque lesdits moyens d'actionnement (14) sont commandés, lesdits moyens de liaison (7) à extrémité fendue occupent la position volontairement écartée pour permettre l'introduction dudit fil (8) dans ledit passage présentant alors un diamètre supérieur à celui dudit fil, et lorsque lesdits moyens d'actionnement (14) sont relâchés, lesdits moyens de liaison (7) occupent la position spontanément rapprochée, pour permettre le serrage par pincement de l'extrémité proximale (8A) du fil (8).

4. Dispositif selon l'une des revendications 2 ou 3,
caractérisé en ce que ledit organe (6) présente une forme cylindrique de révolution, et en ce que ledit passage (11) est situé axialement dans ledit organe, depuis la face transversale (6C) de son extrémité libre, tandis que ladite fente longitudinale (11) débouche au droit dudit passage.

5. Dispositif selon l'une des revendications 2 à 4,
caractérisé en ce que ladite fente longitudinale (12) traverse de part en part l'extrémité libre (6A) dudit organe (6) en passant par ledit passage, pour la séparer en deux parties identiques (6E).

6. Dispositif selon l'une quelconque des revendications précédentes 1 à 5,
caractérisé en ce qu'il comprend, de plus, un corps creux (9) entourant ledit organe (6) et portant, avantageusement, à l'une de ses extrémités à l'aplomb desdits moyens de liaison, lesdits moyens d'actionnement (14).

7. Dispositif selon la revendication 6,
caractérisé en ce que lesdits moyens d'actionnement (14) comprennent au moins une pièce d'écartement déplaçable (15) qui est logée dans un trou (9D) prévu radialement dans l'extrémité correspondante (9A) dudit corps creux, et qui fait face à ladite fente (12), ladite pièce (15) étant susceptible de pénétrer dans ladite fente pour écarter les faces latérales (6F) qui la délimitent et agrandir ledit passage, lorsqu'elle est actionnée.

8. Dispositif selon les revendications 5 et 6,
caractérisé en ce que, lorsque ladite fente longitudinale (12) traverse diamétralement de part en part l'extrémité libre (6A) dudit organe, lesdits moyens d'actionnement (14) comprennent, en regard des bords latéraux (6H) respectivement opposés délimitant ladite fente, une pièce d'écartement déplaçable (15) et une pièce d'écartement fixe (16), lesdites pièces étant logées respectivement dans des trous radiaux opposées (9D,9E), prévus dans l'extrémité correspondante dudit corps creux.

9. Dispositif selon la revendication 7 ou 8,
caractérisé en ce que, entre l'extrémité libre (6A) dudit organe et l'extrémité correspondante (9A) dudit corps, est prévu un manchon d'amortissement, en élastomère ou analogue (17), traversé par la ou lesdites pièces d'écartement.

10. Dispositif selon l'une quelconque des revendications 7 à 9,
caractérisé en ce que ladite pièce d'écartement (15) est radialement déplaçable en direction de la fente (12) par une vis de pression (18) qui est logée dans ledit corps pour agir sur ladite pièce, et qui est commandable par une poignée ou analogue (19) extérieure audit corps.

11. Dispositif selon l'une quelconque des revendications 7 à 10,
caractérisé en ce que la ou lesdites pièces (15,16) d'écartement sont terminées par des pointes coniques (15A,16A) susceptibles de s'engager dans des empreintes (6I) sensiblement complémentaires, ménagées dans les bords latéraux (6H) délimitant ladite fente (12).

12. Dispositif selon la revendication 11,
caractérisé en ce qu'autour de la ou desdites pièces d'écartement, sont disposés des joints toriques ou analogues (20) entre celles-ci et la surface latérale externe (6D) de ladite extrémité libre dudit organe (6).

## Claims

1. Ultrasonic percussion device, of the type including:
- an ultrasonic wave generator (2), terminated by an unit (6) transmitting the vibration generated;
- a wire (8), the near end of which is carried by said unit and the far end of which is set into percussive movement; and
- linking means (7) for immobilizing the near end of said wire in said generator unit,
characterized in that said linking means (7) form an integral part of said unit (6), in that said unit (6) and said linking means (7) are produced from an elastically deformable material and in that said linking means (7) are capable of occupying a spontaneously close-together position under the action of the characteristic elasticity of said elastically deformable material, a close-together position for which the near end (8A) of said wire (8) is clamped by said unit, and a position deliberately spaced apart by actuation means (14), counter to the action of the characteristic elasticity of said elastically deformable material, a spaced-apart position for which the near end (8A) of said wire may be freed from said unit or engaged into said unit.

2. Device according to Claim 1, characterized in that said linking means (7) form part of the free end (6A) of said unit, opposite its other end linked to said generator, and they are defined by a passage (11), formed in said free end of said unit from its transverse face, and able to accommodate the near end of said wire, and by at least one longitudinal slot (12) formed at the end of said free end, from its outer lateral surface (6D) up to said passage (11), in order to shape it into an elastically deformable slotted end.

3. Device according to Claim 2, characterized in that said passage (11) initially exhibits a diameter less than the diameter of said wire, in such a way that, when said actuation means (14) are operated, said linking means (7) with slotted end occupy the deliberately spaced-apart position in order to allow said wire (8) to be inserted into said passage then exhibiting a diameter greater than that of said wire, and, when said actuation means (14) are released, said linking means (7) occupy the spontaneously close-together position, in order to allow clamping of the near end (8A) of the wire (8) by pinching.

4. Device according to either of Claims 2 and 3, characterized in that said unit (6) exhibits an axisymmetric cylindrical shape, and in that said passage (11) is situated axially in said unit, from the transverse face (6C) of its free end, while said longitudinal slot (11) opens out in line with said passage.

5. Device according to one of Claims 2 to 4, characterized in that said longitudinal slot (12) traverses the free end (6A) of said unit (6) from end to end, passing through said passage, so as to separate it into two identical parts (6E).

6. Device according to any one of the preceding Claims 1 to 5, characterized in that it moreover comprises a hollow body (9) surrounding said unit (6) and advantageously bearing said actuation means (14) at one of its ends vertically in line with said linking means.

7. Device according to Claim 6, characterized in that said actuation means (14) comprise at least one movable spacing piece (15) which is lodged in a hole (9D) provided radially in the corresponding end (9A) of said hollow body, and which faces said slot (12), said piece (15) being capable of penetrating into said slot in order to space apart the lateral faces (6F) which delimit it and to enlarge said passage, when it is actuated.

8. Device according to Claims 5 and 6, characterized in that, when said longitudinal slot (12) diametrally traverses the free end (6A) of said unit from end to end, said actuation means (14) comprise, facing respectively opposed lateral edges (6H) delimiting said slot, a movable spacing piece (15) and a fixed spacing piece (16), said pieces being lodged respectively in opposed radial holes (9D, 9E), provided in the corresponding end of said hollow body.

9. Device according to Claim 7 or 8, characterized in that, between the free end (6A) of said unit and the corresponding end (9A) of said body, a damping sleeve is provided, made of elastomer or the like (17), traversed by the spacing piece or pieces.

10. Device according to any one of Claims 7 to 9, characterized in that said spacing piece (15) is radially movable toward the slot (12) by a pressure screw (18) which is lodged in said body in order to act on said piece, and which can be operated by a handle or the like (19) external to said body.

11. Device according to any one of Claims 7 to 10, characterized in that said spacing piece or pieces (15, 16) are terminated by conical points (15A, 16A) capable of engaging in substantially complementary imprints (6I) formed in the lateral edges (6H) delimiting said slot (12).

12. Device according to Claim 11, characterized in that, around said spacing piece or pieces, o-rings or the like (20) are arranged between the pieces and the outer lateral surface (6D) of said free end of said unit (6).

## Patentansprüche

1. Ultraschallperkussionsgerät mit:
- einem Ultraschallgenerator (2) mit einem Abschlußorgan (6) zur Übertragung der erzeugten Schwingungen;
- einem Draht (8), dessen proximales Ende sich am Organ befindet und dessen distalem Ende eine Perkussionsbewegung aufgegeben wird; und
- Verbindungsmitteln (7) zur Befestigung des proximalen Endes des Drahtes im Übertragungsorgan des Generators,
dadurch gekennzeichnet, daß die Verbindungsmittel (7) Bestandteil des Übertragungsorgans (6) sind, dadurch, daß das Übertragungsorgan (6) und die Verbindungsmittel (7) aus einem elastisch verformbaren Material hergestellt sind und dadurch, daß die Verbindungsmittel (7) eine durch die Eigenelastizität des elastisch verformbaren Materials selbsttätig angestellte Stellung, in der das proximale Ende (8A) des Drahtes (8) durch das Organ geklemmt ist, und eine durch Betätigungsmittel (14) entgegen der Eigenelastizität des elastisch verformbaren Materials bewußt abgestellte Stellung einnehmen können, in der das proximale Ende (8A) des Drahtes aus dem Organ entfernt oder in dieses eingeführt werden kann.

2. Gerät nach Anspruch 1,
dadurch gekennzeichnet, daß die Verbindungsmittel (7) Bestandteil des freien Endes (6A) des Organs sind, das dem anderen, mit dem Generator verbundenen Ende entgegengesetzt ist, und daß sie aus einer Durchführung (11), die im freien Ende des Organs von dessen Querseite aus angebracht ist und das proximale Ende des Drahtes aufnehmen kann, und aus mindestens einem Längsspalt (12) bestehen, der am Schluß des freien Endes von dessen äußerer Seitenfläche (6D) aus bis zur Durchführung (11) angebracht ist und dieses in ein elastisch verformbares Spaltende verwandelt.

3. Gerät nach Anspruch 2,
dadurch gekennzeichnet, daß die Durchführung (11) anfangs einen kleineren Durchmesser als der Draht aufweist, so daß, wenn die Betätigungsmittel (14) betätigt werden, die Verbindungsmittel (7) mit Spaltende die bewußt abgestellte Stellung einnehmen, um den Draht (8) in die Durchführung einführen zu können, die dann einen größeren Durchmesser als der Draht hat, und, wenn die Betätigungsmittel (14) losgelassen werden, die Verbindungsmittel (7) die selbsttätig angestellte Stellung zum Klemmen des proximalen Endes (8A) des Drahtes (8) einnehmen.

4. Gerät nach einem der Ansprüche 2 oder 3,
dadurch gekennzeichnet, daß das Organ (6) die Form eines Rotationszylinders hat, und dadurch, daß die Durchführung (11) axial im Organ von der Querseite (6C) des freien Endes aus angeordnet ist, während der Längsspalt (11) rechtwinklig zur Durchführung mündet.

5. Gerät nach einem der Ansprüche 2 bis 4,
dadurch gekennzeichnet, daß der Längsspalt (12) durch das freie Ende (6A) des Organs (6) über die Durchführung verläuft und dieses in zwei identische Teile (6E) teilt.

6. Gerät nach einem der obigen Ansprüche 1 bis 5,
dadurch gekennzeichnet, daß es außerdem ein hohles Gehäuse (9) um das Organ (6) herum hat, an dessen einem Ende sich vorteilhafterweise rechtwinklig zu den Verbindungsmitteln die Betätigungsmittel (14) befinden.

7. Gerät nach Anspruch 6,
dadurch gekennzeichnet, daß die Betätigungsmittel (14) mindestens ein verstellbares Abstellteil (15) haben, das sich in einer Bohrung (9D) radial im entsprechenden Ende (9A) des hohlen Gehäuses gegenüber dem Spalt (12) befindet, wobei das Teil (15) bei Betätigung in den Spalt eindringen kann, um dessen Seiten (6F) abzustellen und die Durchführung zu vergrößern.

8. Gerät nach den Ansprüchen 5 und 6,
dadurch gekennzeichnet, daß, wenn der Längsspalt (12) diametral durch das freie Ende (6A) des Organs verläuft, die Betätigungsmittel (14) gegenüber den einander entgegengesetzten seitlichen Rändern (6H) des Spalts ein verstellbares Abstellteil (15) und ein festes Abstellteil (16) haben, die jeweils in einander entgegengesetzten radialen Bohrungen (9D,9E) im entsprechenden Ende des hohlen Gehäuses angeordnet sind.

9. Gerät nach Anspruch 7 oder 8,
dadurch gekennzeichnet, daß zwischen dem freien Ende (6A) des Organs und dem entsprechenden Ende (9A) des Gehäuses eine Dämpfungsmuffe aus Elastomer oder einem ähnlichen Material (17) vorgesehen ist, durch die das oder die Abstellteile verlaufen.

10. Gerät nach einem der Ansprüche 7 bis 9,
dadurch gekennzeichnet, daß das Abstellteil (15) radial in Richtung des Spalts (12) durch eine Klemmschraube (18) verstellbar ist, die sich zur Einwirkung auf das Teil im Gehäuse befindet und mit einem Griff oder ähnlichem (19) außerhalb des Gehäuses betätigt werden kann.

11. Gerät nach einem der Ansprüche 7 bis 10,
dadurch gekennzeichnet, daß das oder die Abstellteile (15,16) in konischen Spitzen (15A,16A) enden, die in annähernd komplementäre Vertiefungen (6I) in den Seitenrändern (6H) des Spalts (12) eingreifen können.

12. Gerät nach Anspruch 11,
dadurch gekennzeichnet, daß um das oder die Abstellteile herum zwischen denselben und der äußeren Seitenfläche (6D) des freien Endes des Organs (6) Rundringdichtungen oder ähnliche (20) angebracht sind.
